# EUROPÄISCHE PATENTSCHRIFT

(11) **EP 3 666 229 B1**
(45) Veröffentlichungstag und Bekanntmachung des Hinweises auf die Patenterteilung: **17.09.2025**
(21) Anmeldenummer: 18211379.5
(22) Anmeldetag: 10.12.2018
(51) Int. Cl.: A61F 2/38, A61F 2/46, A61B 17/15, A61F 2/30, A61B 90/00

(54) **KNIEGELENKENDOPROTHESEN-SATZ UND INSTRUMENTARIUM**
KNEE JOINT ENDOPROSTHETIC SET AND INSTRUMENTS
ENSEMBLE D'ENDOPROTHÈSES DE L'ARTICULATION DU GENOU ET INSTRUMENT

(43) Veröffentlichungstag der Anmeldung: 17.06.2020
(73) Patentinhaber: Waldemar Link GmbH & Co. KG, 22339 Hamburg (DE)
(72) Erfinder: Jensen, Harm-Iven, 24214 Noer (DE); Link, Helmut D., 22397 Hamburg (DE)
(74) Vertreter: Glawe, Delfs, Moll

(56) Entgegenhaltungen:
- EP-A1- 0 410 237
- WO-A1-98/42279
- DE-A1- 10 036 636
- US-A- 5 342 366
- US-A- 5 464 406
- US-A1- 2006 142 778
- US-B1- 6 254 640
- US-B1- 6 673 077

## Beschreibung

Die Erfindung betrifft einen Satz von Kniegelenkendoprothesen, und zwar speziell die femoralen Komponenten der Kniegelenkendoprothesen. Die femorale Komponente einer Kniegelenkendoprothese wirkt zusammen mit einer tibialen Komponente; diese beiden Komponenten bilden im Wesentlichen die Kniegelenkendoprothese. Der Satz umfasst Kniegelenkendoprothesen in verschiedenen Größen.

Kniegelenkendoprothesen zum totalen Ersatz eines natürlichen Kniegelenks sind bereits seit langem bekannt. Typischerweise weist eine Kniegelenkendoprothese eine femorale Komponente sowie eine tibiale Komponente auf, die bei der Implantation vom Chirurgen am distalen Ende eines Femurs und entsprechend am proximalen Ende der Tibia eingesetzt werden. Die femorale Komponente wirkt mit der tibialen Komponente derart zusammen, dass sie die Scharnierfunktion des natürlichen Kniegelenks nachbildet. Dazu weisen die beiden Komponenten in der Regel jeweils ein Gelenksstück und einen Schaft zur Verankerung auf. Derartige Knieprothesen werden auch als Total-Knieprothesen bezeichnet.

Eine solche Kniegelenk-Endoprothese ist beispielsweise offenbart in EP 0 410 237 A1. Sie umfasst ein Femurteil mit einem Schaft zur Verankerung im medullären Kanal des Femurs. Der Schaft verjüngt sich zu seinem proximalen Ende hin.

Bei Hüftgelenk-Endoprothesen ist es bekannt, einen Prothesensatz mit Prothesen jeweils umfassend einen Schaft in unterschiedlichen Größen bereitzustellen (WO 98/42279 A1). Hierbei ist der Schaft im proximalen Bereich im Querschnitt oval und verjüngt sich zum distalen Ende hin zu einem kreisrunden Querschnitt. Für unterschiedliche Größen wird der Schaft im wesentlichen formgleich proportional vergrößert/verkleinert.

Zur Anpassung an unterschiedliche anatomische Verhältnisse bei den Patienten sind Kniegelenkendoprothesen in mehreren Größen erhältlich. Bei serienmäßig hergestellten modularen Kniegelenkendoprothesen sind die Komponenten zwar vergleichsweise kostengünstig in verschiedenen Größen erhältlich, jedoch ist die Anpassung an die jeweiligen anatomischen Verhältnisse häufig nicht befriedigend. Es kann daher erforderlich sein, die Prothese mit Zement in dem medullären Kanal des Femurs einzubetten und zu fixieren. Dies bringt jedoch häufig den Nachteil einer unzureichenden Langzeitstabilität mit sich. Es kann zu Lockerungen im Zement kommen, wodurch der Schaft der femoralen Komponente nicht mehr sicher im Femur verankert ist. In vielen Fällen, besonders bei kondylärem Knochenverlust, ist der Schaft in der Regel entscheidend für die Lastübertragung und wesentlich für die Führung der Prothese. Bei einer Lockerung kann er keiner dieser Funktionen mehr genügen, sodass die Prothese ausfällt. Eine Revisionsoperation ist dann erforderlich. - Durch individuelle Anfertigung des Schafts zur Anpassung an die jeweiligen anatomischen Verhältnisse kann dieser Problematik begegnet werden. Dies kann erfolgen nach einem CT-Scan des Femurs, um so die individuelle Form des medullären Kanals zu bestimmen und danach den Schaft zu formen. Es handelt sich hierbei dann aber um eine Sonderanfertigung. Dies hat zwar den Vorteil einer guten Passform und damit hoher Langzeitstabilität, ist aber sehr aufwendig.

Der Erfindung liegt die Aufgabe zugrunde, die Vorteile der beiden verschiedenen Ansätze miteinander zu verknüpfen.

Die erfindungsgemäße Lösung liegt in den Merkmalen des unabhängigen Anspruchs.

Vorteilhafte Weiterbildungen sind Gegenstand der abhängigen Ansprüche.

Bei einem Kniegelenkendoprothesen-Satz umfassend modulare Kniegelenkendoprothesen in verschiedenen Größen, wobei eine jeweilige Kniegelenkendoprothese umfasst eine tibiale Komponente zur Verankerung an einem proximalen Ende der Tibia, eine femorale Komponente zur Verankerung an einem distalen Ende des Femurs, wobei die femorale Komponente ein Gelenkelement zum gelenkigen Zusammenwirken mit der tibialen Komponente sowie einen Schaft ausgebildet zur Verankerung in einem Medullärkanal des Femurs umfasst, wobei der Satz Schäfte in verschiedenen Größen umfasst, ist erfindungsgemäß vorgesehen, dass bei der femoralen Komponente eine Form der Schäfte so gewählt ist, dass sie sich von einem distalen, dem Gelenkelement zugewandten Ende zu einem proximalen, freien Schaftende hin verjüngt und an dem distalen Ende mit einem ovalen Querschnitt und an dem proximalen, freien Schaftende mit einem runden Querschnitt ausgeführt ist, wobei eine durch den ovalen Querschnitt bestimmte Ovalität bezüglich ihres Ovalitätsgrads mit zunehmender Größe der Schäfte ansteigt, wobei der Ovalitätsgrad der Ovalität bestimmt ist durch ein Verhältnis von ihrer längeren zu ihrer kürzeren Achse.

Die Erfindung hat erkannt, dass durch eine im Querschnitt veränderliche Gestaltung des Schafts in der beanspruchten Weise eine besonders gute Anpassung der verschiedenen Größen an die anatomischen Verhältnisse des Femurs und seines Markkanals erreicht werden kann. Die ovale Form am distalen Ende in Kombination mit dem Übergang zu einer runden Gestaltung am freien Ende ergibt einen rotationssicheren und festen Sitz der femoralen Komponente im Knochen. Der Schaft wird damit auf vorteilhafte Weise lasttragend und nimmt nicht nur, wie im Stand der Technik überwiegend, nur eine Führungsfunktion ein, sondern gewährleistet auch die Halte- und Befestigungsfunktion. Dieser Vorteil kann dazu genutzt werden, einen Satz zu bilden, wobei mit in wenigen vorbestimmten Größen serienmäßig hergestellten Schäften eine vergleichbar sichere Verankerung erzielt werden kann, wie sie bisher überwiegend Sonderanfertigungen - angepasst an die jeweilige Anatomie des Patienten - vorbehalten waren. Damit ergibt sich eine deutlich bessere Verträglichkeit und Langzeitstabilität der Prothesen. Das Risiko von komplikationsprächtigen Revisionsoperation wird gesenkt, besonders auch für adipöse Patienten, die erfahrungsgemäß besonders dem Risiko einer Implantatlockerung ausgesetzt sind.

Es ist ein Verdienst der Erfindung erkannt zu haben, dass es die Ovalität mit ihrem Ovalitätsgrad ist, die entscheidend ist für die Größenstaffelung der Schäfte. Sie wendet sich damit ab von den bisherigen Ansätzen, welche typischerweise die Schaftlänge als einen entscheidenden Parameter für die Größe zugrunde gelegt haben. Dass damit ein besonders guter Sitz erreicht werden kann mit einer überschaubaren Anzahl von Größen, ist vollkommen überraschend.

Nachfolgend seien zuerst einige verwendete Begriffe erläutert:
Unter einer ML-Richtung wird eine von medial nach lateral verlaufende Richtung verstanden. Sie läuft quer zur Sagittalebene eines Körpers. Es handelt sich also um eine Transversalrichtung. Sie wird in der Anatomie auch als Querachse bezeichnet und entspricht somit weitgehend (wenn auch nicht unbedingt ganz genau) der Achse für die Beugebewegung des Kniegelenks.

Unter einem ML-Maß wird eine Abmessung in ML-Richtung verstanden, beispielsweise eine Ausdehnung der Ovalität in ML-Richtung.

Unter einer AP-Richtung wird eine von anterior nach posterior, also von der Vorderseite zur Rückseite des Körpers verlaufende Richtung verstanden. Diese Richtung ist quer zur Frontalebene des Körpers. Sie wird in der Anatomie auch als Pfeilachse bezeichnet und steht senkrecht auf der Sagittalebene.

Unter einem AP-Maß wird eine Abmessung in AP-Richtung verstanden, beispielsweise eine Ausdehnung der Ovalität in AP-Richtung. Sämtliche vorgenannten Richtungsangaben beziehen sich auf den eingebauten (implantierten) Zustand der Endoprothese.

Eine Ovalität wird bestimmt durch ihren ovalen Querschnitt. Eine Größe der Ovalität ist abhängig von der Größe des Querschnitts. Ein Ovalitätsgrad einer Ovalität ist, wie angegeben, gebildet durch ein Verhältnis von ihrer längeren zu ihrer kürzeren Achse, beispielsweise durch das Verhältnis zwischen ihrem ML-Maß und ihrem AP-Maß. Je größer dieses Verhältnis wird, desto größer ist der Ovalitätsgrad und desto größer die Abweichung von der Kreisform.

Unter einem runden Querschnitt wird eine im Wesentlichen kreisrunde Ausführung verstanden.

Unter einem äquivalenten Durchmesser wird bei kreisrunder Form der Durchmesser und bei nicht kreisrunder Form ein mittlerer Durchmesser verstanden. Ein eventuelles Untermaß, wie es bei zur zementierten Implantation ausgebildeten Schäften vorkommen kann im Vergleich zur zementfreien Implantation ausgebildeten Schäften gleicher Größe, wird bei der Bestimmung des äquivalenten Durchmessers nicht berücksichtigt.

Gemäß der Erfindung ist eine Gestaltung der Ovalität vorgesehen, bei der der Ovalitätsgrad zwischen den Schäften unterschiedlicher Größe verschieden ist, und zwar derart, dass der Ovalitätsgrad mit zunehmender Größe der Schäfte ansteigt. Hierbei wird die überraschende Erkenntnis ausgenutzt, dass es vorteilhaft ist, die Ovalität in definierter Weise über die verschiedenen Größen hinweg zu ändern. Dies geschieht zweckmäßigerweise so, dass die Ovalität zwischen den verschiedenen Größen nicht formgleich ist (und sich dann nur in Bezug auf ihre Größe unterscheidet), sondern dass die Ovalität planvoll formvariabel sein soll. Es ist ein Verdienst der Erfindung erkannt zu haben, dass es insbesondere der Ovalitätsgrad ist, der eine ganz hervorragende Korrelation zu den verschiedenen Größen des Schafts bietet. Mit dieser Gestaltung wendet sich die Erfindung ab von den bisherigen Ansätzen, welche ganz überwiegend die Schaftlänge als den entscheidenden Parameter für die Größe zugrunde gelegt haben. Auch die reine Schaftdicke wird nicht als entscheidender Parameter für die Größe verwendet. Der Stand der Technik enthält keinerlei Hinweis, einen Satz von Schäften verschiedener Größen für Kniegelenkendoprothesen zu schaffen, wobei jede Größe einen anderen, eigenen Ovalitätsgrad aufweist. Dass damit ein besonders guter Sitz erreicht werden kann mit einer überschaubaren Anzahl von Größen, ist vollkommen überraschend.

Vorzugsweise weist der ovale Querschnitt eine solche Ovalität auf, dass deren größere Achse in ML-Richtung und deren kleinere Achse in AP-Richtung liegt, wobei vorzugsweise ein Verhältnis der langen Achse zur kurzen Achse im Bereich zwischen 1,1 und 1,4 liegt. Mit dieser speziellen Konfiguration der Ovalität in dem überraschend schmalen Band des Achsenverhältnisses kann, wie die Erfindung erkannt hat, ein überragend guter Sitz erreicht werden. Dies kann weiter gesteigert werden, indem die Ovalität ellipsenartig ausgeführt wird.

Die Kniegelenkendoprothese ist modular aufgebaut, wobei Schäfte von verschiedenen Größen vorgesehen sind, die wahlweise mit der Gelenkkomponente verbindbar sind.

Vorzugsweise weisen die Schäfte jeweils eine Mantelfläche auf, die zur Anlage an eine Innenwandung des medullären Kanals ausgebildet ist. Zusätzlich oder alternativ können die Schäfte jeweils eine solche Mantelfläche aufweisen, die einem konischen Übergangskörper zwischen einem ovalen, insbesondere in ellipsenförmigen, Querschnitt an einem und einem kreisförmigen Querschnitt am anderen Ende entspricht. Damit kann eine großflächige Anlage erreicht werden, die zu einer günstigen Lasteinleitung in den Knochen und Druckverteilung an dem Knochen führt. Die Tragfähigkeit sowie die Langzeitstabilität können damit verbessert werden.

Die Schäfte sind so ausgebildet, dass sie, insbesondere wenn zementfrei aus Titan ausgeführt, ein E-Modul im Bereich von 70 bis 120 GPa aufweisen. Damit liegen sie im physiologisch günstigen Bereich und können zum einen eine gute Kraftübertragung auf den Knochen gewährleisten und zum anderen eine Degeneration des Knochens verhindern, wie sie bei ansonsten unpassendem E-Modul leicht auftreten könnte (Wolffsches-Transformationsgesetz).

Ferner können die Schäfte mit Vorteil gekrümmt ausgeführt sein, und zwar sind sie schwach gekrümmt mit einer Krümmung, die einen Krümmungsradius von mindestens 1000 mm aufweist. Mit einer solchen verhältnismäßig schwachen Krümmung kann eine eindeutige Positionierung des Schafts und damit der Kniegelenkendoprothese im Ganzen im Knochen erreicht werden. Die Erfindung macht sich hierbei zu Nutze, dass typischerweise der medulläre Kanal des Femurs nicht ganz gerade ist, sondern eine leichte Krümmung aufweist. Indem der Schaft ebenfalls eine Krümmung aufweist, entsteht somit eine Vorzugsposition. Dies bedeutet für den Operateur, dass der Schaft sich sozusagen selbst positioniert. Dies sichert nicht nur eine verbesserte Anlage und damit Kraftübertragung zwischen Schaft und Knochen, sondern auch eine positionstreue Implantation. Vorzugsweise sind eventuelle Krümmungen so ausgeführt, dass sie in AP-Richtung und ML-Richtung verschieden groß sind. Besonders bevorzugt ist es, wenn die Schäfte in AP-Richtung stärker gekrümmt sind. Dies kann sogar so weit gehen, dass die Schäfte in ML-Richtung gar keine Krümmung aufweisen, also ungekrümmt sind. Insbesondere sind die Schäfte vorzugsweise eindimensional gekrümmt; somit gibt es nur eine Krümmungsebene. Damit ergibt sich eine verhältnismäßig einfache Grundform, die rationell herstellbar ist und dennoch eine gute dauerhafte Befestigung ermöglicht.

Günstig ist eine Ausführung der Schäfte als Kurzschäfte mit einer Länge von weniger als dem 7-fachen eines äquivalenten Durchmessers des Schafts am distalen Ende. Ein solcher Kurzschaft bietet den Vorteil, dass er zum einen einen verhältnismäßig großen Konuswinkel aufweist und somit eine universellere Anpassbarkeit ermöglicht; zum anderen bietet dies den für die erfolgreiche Implantation und die Gesundheit des Patienten besonders wertvollen Vorteil, dass der Kurzschaft weniger tief in den Knochen eindringt und somit die Gefahr eines tiefen Einbringens von Bakterien oder anderen Keimen in den Knochen verringert wird. Zweckmäßigerweise werden die Kurzschäfte daher noch kürzer ausgeführt, und zwar vorzugsweise mit einer Länge die weniger als dem Fünffachen, weiter vorzugsweise jedoch mehr als dem Zweifachen des äquivalenten Durchmessers am distalen Schaftende entspricht. Damit wird die Eindringtiefe der Prothese weiter verringert, wobei die Mindestlänge sicherstellt, dass immer noch eine ausreichend große Fläche zur Kraftübertragung zur Verfügung steht und eine ausreichende Führung gewährleistet ist.

Das freie Ende am Schaft ist zweckmäßigerweise domförmig verrundet ausgeführt. Eine solche, etwa einer halbsphärischen Gestaltung ähnelnde Schaftspitze ermöglicht ein vereinfachtes Einführen und Einsetzen des Schafts in den medullären Kanal des Femurs. Ferner wirkt eine solche Gestaltung atraumatisch und schont das empfindliche Innere des Knochens. Besonders bewährt ist es, wenn das freie Ende rundum verrundet ausgeführt ist.

Gemäß einer weiteren besonders vorteilhaften Ausführungsform der Erfindung sind Adapter vorgesehen, die das Gelenkelement mit einem der Schäfte verbinden, wobei vorzugsweise Adapter in verschiedenen Längen vorgesehen sind. Zweckmäßig ist eine Ausführung der Adapter als Steckadapter. Auf diese Weise kann durch Wahl eines passenden Adapters eine Längenveränderung erreicht werden, ohne dass dazu ein anderer Schaft benötigt würde. Somit kann auf einfache Weise und ohne zusätzliche Größen für den erfindungsgemäßen Kniegelenkendoprothesen-Satz zu erfordern, eine präzisere Anpassung an die jeweiligen anatomischen Gegebenheiten des Patienten erreicht werden. Die Adapter sind mit Vorteil winkelverstellbar ausgeführt, und zwar so, dass sie in ihrer Winkelposition arretierbar sind. Damit kann eine definierte relative Verdrehung zwischen Schaft und Gelenkelement eingestellt und gesichert werden. Dies verbessert ebenfalls die Anpassungsbarkeit des erfindungsgemäßen Kniegelenkendoprothesen-Satzes an die jeweiligen anatomischen Verhältnisse des Patienten, ohne dass zusätzliche Schaftmodelle für den Satz erforderlich wären. Zweckmäßig ist dazu der Adapter als ein Doppelkonus ausgeführt oder mit einer Vielfach-Verzahnung versehen. Letztere bietet den Vorteil einer formschlüssigen Winkelverstellung, während erstere den Vorteil einer stufenlosen Verstellbarkeit des Winkels bietet.

Der Kniegelenkendoprothesen-Satz umfasst zweckmäßigerweise sowohl Schäfte zur Befestigung mittels Zement wie auch Schäfte zur zementfreien Befestigung. Damit kann flexibel auf verschiedene Anforderungen reagiert werden. Hierbei weisen die Schäfte zur Befestigung mittels Zement vorzugsweise ein vorbestimmtes Untermaß bezogen auf die korrespondierenden Schäfte zur zementfreien Befestigung auf. Damit ist es ermöglicht, dass quasi größengleich zementfreie gegen zu zementierende Schäfte ausgetauscht werden können, und zwar erforderlichenfalls auch noch intraoperativ. Das Anwendungsspektrum des erfindungsgemäßen Kniegelenkendoprothesen-Satzes erweitert sich damit beträchtlich. Hierbei kann vorgesehen sein, dass die Schäfte zur Befestigung mittels Zement eine glatte Mantelfläche aufweisen, die gegebenenfalls mit einigen wenigen Furchen (maximal 5) versehen ist, wohingegen die Schäfte zur zementfreien Befestigung vorzugsweise eine geriffelte Mantelfläche aufweisen. Typischerweise sind bei einer Riffelung der Mantelfläche mindestens 16, vorzugsweise mindestens 20 Riffelleisten über den Umfang des Schafts in axialer Richtung erstreckend angeordnet. Durch die Riffelung wird insbesondere die initiale Befestigungssicherheit bei zementfreier Implantation erhöht, und durch die glatte Gestaltung der Mantelfläche bzw. durch die wenigen Furchen kann entsprechend eine Verbesserung einer zementierten Befestigung erreicht werden.

Größenmäßig sind die Schäfte vorzugsweise gestaffelt nach dem ML-Maß, und zwar regelmäßig. Bewährt hat sich eine Abstufung in regelmäßigen Schritten. Die Regelmäßigkeit kann beispielsweise durch eine Reihe begründet sein, insbesondere eine lineare Reihe, eine logarithmische Reihe oder eine geometrische Reihe. Besonders bevorzugt ist eine regelmäßige Staffelung auf Basis eines Modulmaßes. Das Modulmaß (a) entspricht einem Größenschritt zwischen zwei unmittelbar aufeinanderfolgenden Größen; hierbei erfolgt auch die Festlegung der kleinsten und größten Größe des Satzes auf Basis des Modulmaßes. Dieses kann beispielsweise in der Weise geschehen, dass die kleinste Größe etwa einem 10- bis 15-fachen des Modulmaßes a (beispielsweise 13·a) und die größte Größe etwa einem 20- bis 30-fachen des Modulmaßes a (beispielsweise 23·a) entspricht. So kann durch die Festlegung von nur einem Maß, nämlich des Modulmaßes a, eine zweckmäßige Staffelung und damit Auswahl der Größen für die Schäfte des Satzes erreicht werden. Besonders bevorzugt ist, wenn die Schäfte sich etwa im Größenverhältnis im Bereich 1:2 erstrecken, und das Modulmaß a vorzugsweise so gewählt ist, dass sich zwischen 8 und 14, weiter vorzugsweise zwischen 10 und 12, verschiedene Größen ergeben.

Zum Implantieren einer femoralen Komponente einer Kniegelenkendoprothese aus dem Kniegelenkendoprothesen-Satz kann ein Instrumentarium vorgesehen sein. Wie bereits vorstehend beschrieben, weist hierbei die femorale Komponente der Kniegelenkendoprothese einen Schaft und ein Gelenkelement auf. Das Instrumentarium umfasst ein Werkzeug zur Bildung einer zur Aufnahme des Schafts bemessenen Kavität am distalen Ende des medullären Kanals eines Femurs, eine Lehre zur Herstellung einer Aufnahme für das Gelenkelement am distalen Ende des Femurs, eine Tiefenmesseinrichtung zur Bestimmung einer Positionierung des Schafts in der zur Aufnahme des Schafts geschaffenen Kavität, und ein Einsatzinstrument zum Implantieren der femoralen Komponente am distalen Ende des Femurs, wobei die Tiefenmesseinrichtung ausgebildet ist zur Anzeige einer benötigten Länge des Schafts und/oder eines Adapter zur Befestigung des Schafts am Gelenkelement. Mit diesem Instrumentarium kann mittels der Tiefenmesseinrichtung eine präzise Positionierung des Schafts in dem Knochen erreicht werden. Es versteht sich, dass hierbei die Tiefenmesseinrichtung auf die verschiedenen Größen des Satzes abgestimmt ist. Somit ermöglicht die Tiefenmesseinrichtung dem Operateur eine präzise Positionierung vom Schaft in der Kavität. Die Montagegenauigkeit erhöht sich damit, und der Gefahr von Fehlfunktionen der Kniegelenkendoprothese wird auf wirksame Weise begegnet.

Zusätzlich kann eine Winkelmesseinrichtung vorgesehen sein. Sie ist ausgebildet zur Bestimmung eines Rotationswinkels des Schafts innerhalb des medullären Kanals. Damit kann insbesondere bei einem gekrümmten Schaft, der wie vorstehend beschrieben eine Vorzugsposition im medullären Kanal einnimmt, dessen Winkellage erfasst und bestimmt werden. Dieser Winkel ist bei der Implantation auch für den Schaft der Kniegelenkendoprothese einzustellen, um so einen optimalen Sitz zu erreichen. Da hierbei die Vorzugsrichtung sowohl nach links wie auch nach rechts verdreht sein kann, wobei je nach Körperseite links und rechts abwechselnd für medial und lateral steht, ist zweckmäßigerweise ein gesonderter Indikator für eine Rotationsrichtung des Schafts innerhalb des medullären Kanals vorgesehen. Mit diesem Indikator wird die Gefahr von Verwechslungen von links und rechts bzw. medial und lateral entscheidend verringert, da nur auf den Indikator Bezug genommen und somit geachtet zu werden braucht. Der Indikator kann beispielsweise als eine Stanzmarkierung bzw. ein anderes strukturelles Element an der Winkelmesseinrichtung ausgebildet sein. Mit Vorteil sind die Tiefenmesseinrichtung und die Winkelmesseinrichtung als ein kombiniertes Element ausgeführt. Dies verringert die Anzahl der Teile und vereinfacht die Handhabung.

Ferner kann eine gesonderte Ausrichtlehre vorgesehen sein. Sie ist zweckmäßigerweise am Übergang zwischen Gelenkelement und Schaft anzuordnen, und sie ist dazu ausgebildet, eine relative Rotation zwischen Schaft und Gelenkelement zu bestimmen. Damit kann der von der Winkelmesseinrichtung bestimmte Winkel für eine Verdrehung des Schafts im medullären Kanal kontrolliert werden als relative Rotation zwischen Schaft und Gelenkelement, und zwar vorzugsweise derart, dass eine relative Rotation mittels des Adapters eingestellt wird. Auf diese Weise ist der Schaft dann exakt so ausgerichtet, dass das Gelenkelement korrekt orientiert ist, wenn sich der Schaft in seine Vorzugsposition eingesucht hat. Eine winkelgenaue Montage der Kniegelenkendoprothese wird damit deutlich zuverlässiger und vereinfacht.

Ferner wird eine einzelne Kniegelenkendoprothese aus dem erfindungsgemäßen Kniegelenkendoprothesen-Satz beschrieben, jedoch nicht beansprucht.

Die Erfindung wird nachfolgend unter Bezugnahme auf vorteilhafte Ausführungsformen der Erfindung anhand der Zeichnung beispielhaft beschrieben. Es zeigen:
- Fig. 1: eine perspektivische Ansicht einer Kniegelenkendoprothese im implantierten Zustand am Kniegelenk;
- Fig. 2a, b: schematische Frontal- und Lateraldarstellungen zur femoralen Komponente einer Kniegelenkendoprothese gemäß einem Ausführungsbeispiel;
- Fig. 3a, b: Frontal- und Lateralansicht eines Schafts der femoralen Komponente gemäß dem Ausführungsbeispiel;
- Fig. 4: Querschnittsansicht eines proximalen Schaftendes gemäß einer Linie IV-IV in Fig. 3;
- Fig. 5: Querschnittsansicht eines distalen Schaftendes gemäß einer Linie V-V in Fig. 3;
- Fig. 6a, b: einen Schaft gleicher Größe zur zementfreien bzw. zementierten Implantation;
- Fig. 7: eine Raspel passend zu den Schäften gemäß Fig. 6;
- Fig. 8a, b: perspektivische Ansichten zu den Schäften gemäß Fig. 6a, b;
- Fig. 9: einen Adapter zur Anordnung zwischen Schaft und Gelenkelement der femoralen Komponente;
- Fig. 10a, b, c: Adapter verschiedener Länge kombiniert mit unterschiedlich langen Schäften;
- Fig. 11a, b: eine perspektivische Ansicht zu dem Adapter mit Detaildarstellung;
- Fig. 12a, b: perspektivische Ansichten zu einer kombinierten Tiefen- und Winkelmesseinrichtung;
- Fig. 13: eine Detaildarstellung zur Tiefen- und Winkelmesseinrichtung; und
- Fig. 14a-f: Darstellungen verschiedener Schritte zum Implantieren der Kniegelenkendoprothese gemäß dem Ausführungsbeispiel.

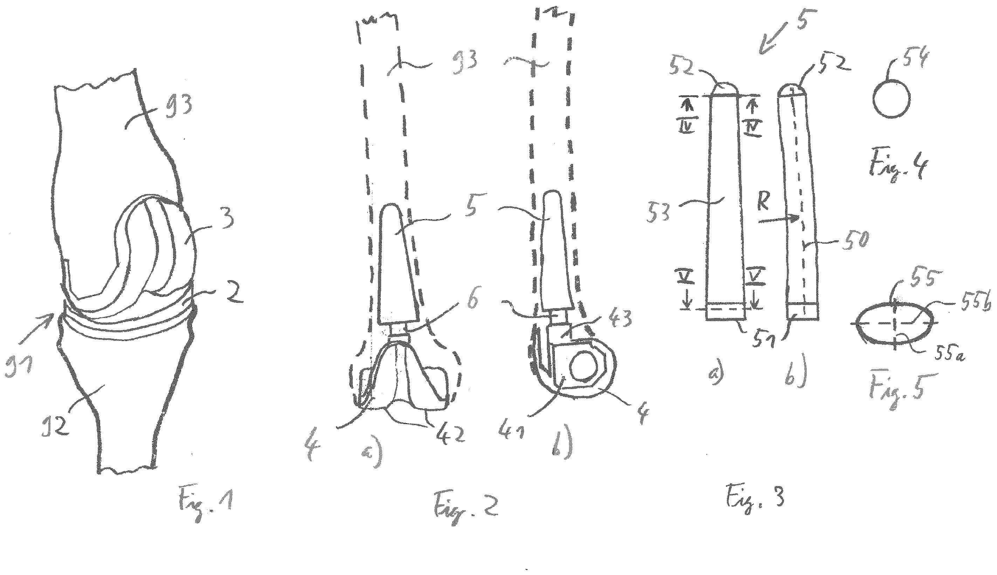

Eine Kniegelenkendoprothese im implantierten Zustand am Knie ist in Figur 1 dargestellt. Es handelt sich um eine Ausschnittdarstellung und zeigt einen Bereich des Oberschenkels um ein Kniegelenk 91. Man erkennt das (obere) proximale Ende einer Tibia 92 sowie das (untere) distale Ende 93 des Femurs. Das natürliche Kniegelenk ist ersetzt durch eine Kniegelenkendoprothese, die eine tibiale Komponente 2 und eine mit ihr gelenkig zusammenwirkende femorale Komponente 3 umfasst.

Die Kniegelenkendoprothese insgesamt sowie ihre tibialen und femoralen Komponenten 2, 3 sind modular aufgebaut. Nachfolgend wird der Aufbau der femoralen Komponente 3 erläutert. Die femorale Komponente 3 ist mit ihren Haupt-Bestandteilen dargestellt in Figur 2, die eine Frontaldarstellung in Figur 2a und eine Ansicht von lateral in Figur 2b zeigt. Die am distalen Ende des Femurs 93 eingesetzte femorale Komponente 3 umfasst als Hauptbestandteil ein Gelenkelement 4, einen Schaft 5 sowie einen Adapter 6. Das Gelenkelement 4 weist nach außen weisende Kondylenelemente 42 zur gelenkigen Interaktion mit der tibialen Komponente 2 auf. Die Kondylenelemente 42 sind angeordnet an einem kastenartigen Hauptkörper 41, der an seinem proximalen Ende ein Koppelstück 43 zur Verbindung mit dem Schaft 5 umfasst.

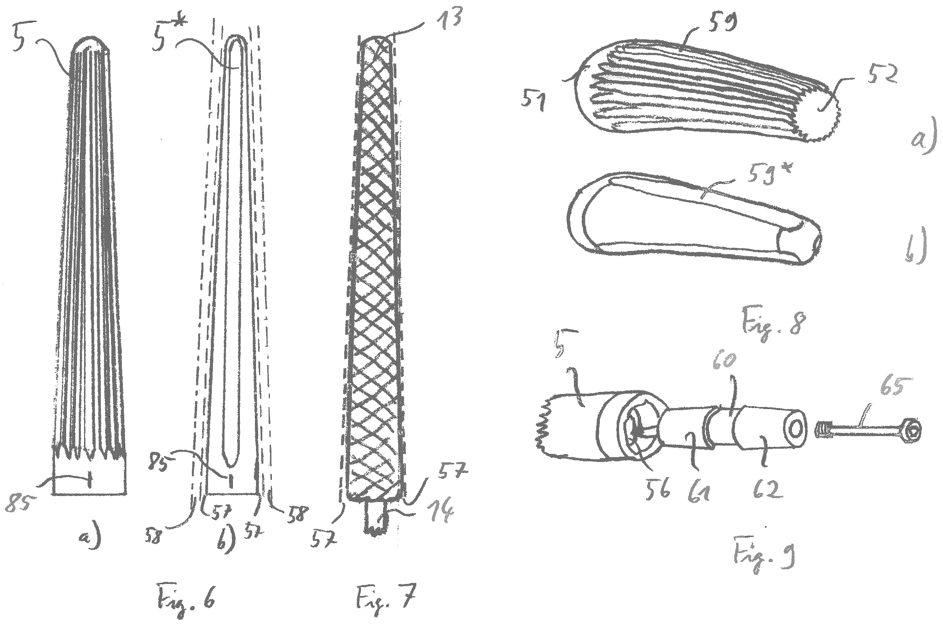

Der Schaft 5 ist über einen Adapter 6 mit dem Gelenkelement 4 verbunden. Bei dem dargestellten Ausführungsbeispiel handelt es sich um ein stiftartigen Adapter 6, der mit einem Doppelkonus versehen ist. Er ist mit seinem distalen Ende in das Koppelstück 43 und mit seinem proximalen Ende in eine entsprechende Aufnahme 56 (siehe Figur 9) am Schaft 5 eingesetzt ist. Der generell konusartig geformte Schaft 5 ist eingesetzt in einen medullären Kanal (Markkanal bzw. Medullärkanal) des Femurs 93, der zur Aufnahme des Schafts 5 entsprechend geweitet ist. Der Schaft 5 kann in dem medullären Kanal 93 gehaltert sein durch einen Presssitz bei zementfreier Implantation oder mit Zement (nicht dargestellt) gesichert sein. Die Implantation und Befestigung einer femoralen Komponente einer Kniegelenkendoprothese als solche sind im Grunde bekannt und brauchen daher nicht näher erläutert zu werden.

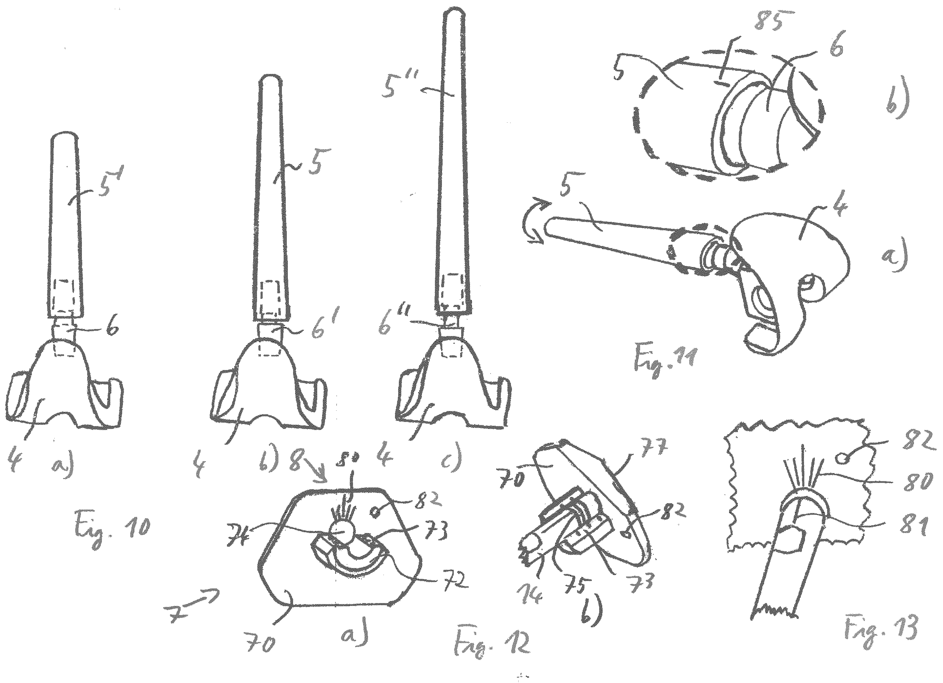

Der Schaft 5 ist modular ausgeführt in verschiedenen Größen. Beispiele für verschiedene Größen des Schafts 5, 5' und 5" sind in Figur 10a, b, c dargestellt. Der erfindungsgemäße Schaft 5 ist in einer besonderen Weise geformt. In Figur 3a ist eine Ansicht des Schafts 5 von frontal dargestellt. Man erkennt eine gerade konische Form mit einem dickeren distalen Ende 51 und einem dünneren proximalen Ende 52.

Das proximale Ende 52 ist verrundet ausgeführt zur Vereinfachung des Einsetzens des Schafts 5 in den medullären Kanal des Femurs 93 und zur Verringerung einer traumatischen Wirkung. Eine Darstellung des proximalen Endes 52 ist in Figur 4 gezeigt. Demnach ist am proximalen Ende 52 der Querschnitt 54 kreisrund. Eine Darstellung des distalen Endes 51 ist als Querschnittsansicht in Figur 5 gezeigt. Demnach ist am distalen Ende 51 der Querschnitt 55 oval, insbesondere elliptisch. Hierbei ist die kürzere Achse 55a in AP-Richtung gelegen und die längere Achse 55b in ML-Richtung. Eine Mantelfläche 53 des Schafts 5 ist somit nicht kegelig, sondern bildet eine Übergangsfläche zwischen einem elliptischen und runden Querschnitt.

In einer Seitenansicht von lateral ist der Schaft 5 ebenfalls konusartig verjüngend ausgeführt, jedoch ist er in dieser Ebene nicht gerade sondern mit einer schwachen Krümmung versehen, wie die in Figur 4b gestrichelt dargestellte Mittellinie 50 visualisiert. Dabei ist ein Krümmungsradius R verhältnismäßig groß, so dass sich eine schwache Krümmung ergibt. Der Krümmungsradius R beträgt in dem dargestellten Ausführungsbeispiel 1500 mm.

Verschiedene Alternativen zur Ausführung des Schafts 5 sind in Figur 6 dargestellt. Sie betreffen insbesondere eine Ausführung des Schafts 5 zur zementfreien Implantation (siehe Figur 6a) und eine Ausführung des Schafts 5* zur zementierten Implantation (siehe Figur 6b). Die beiden Schäfte 5, 5* unterscheiden sich zum einen in Bezug auf die Gestaltung ihrer Mantelfläche 53 und zum anderen in ihrer Weite.

Es wird nun Bezug genommen auf Figur 7, die eine Raspel 13 (oder ein Kompressor) darstellt. Hierbei handelt sich um ein Werkzeug zur Schaffung einer Kavität zur Aufnahme des Schafts 5 in dem Femur 93. Bei der Schaffung der Kavität wird der medulläre Kanal des Femurs 93 insoweit ausgeweitet, dass er zur Aufnahme des Schafts 5 bemessen ist. Dies bezieht sich sowohl auf die Abmessungen hinsichtlich Weite und Tiefe wie auch auf die Krümmung (d. h. die Raspel 13 ist in derselben Weise gekrümmt wie der Schaft 5). Dies geschieht mit großer Präzision, um einen präzisen Sitz des Schafts 5 zu erreichen. Bei dem zur zementfreien Implantation vorgesehenen Schaft 5 bedeutet dies, dass die Kavität nur so weit ausgeweitet wird, dass sich ein Presssitz für den Schaft ergibt. Konkret heißt das, dass das zum Weiten der Kavität verwendete Werkzeug, wie die in Figur 7 dargestellte Raspel 13, eine etwas geringere Weite aufweist als der dazugehörende Schaft 5, nämlich um ein Presssitzmaß 57 (in Figur 7 visualisiert durch die gestrichelte Linie auf jeder Seite der Raspel 13) verringert. Ein Beispiel für ein solches Presssitzmaß ist 0,2 mm auf jeder Seite. Wird beim Implantieren der Schaft 5 in die mit Untermaß geschaffene Kavität eingesetzt, ergibt sich so ein Presssitz, der für eine sichere Verankerung auch ohne Zement sorgt. Zur Steigerung der Befestigungssicherheit ist zweckmäßigerweise vorgesehen, dass die Mantelfläche 53 eine Riffelung aufweist. Die Riffelung ist mit einer Vielzahl von Rillen 59 versehen, in dem dargestellten Ausführungsbeispiel 24 Rillen, wie in Figur 8a dargestellt. Damit ergibt sich ein fester Sitz, und zwar sowohl in Bezug auf eine initiale Befestigung wie auch hinsichtlich einer langzeitstabilen Befestigung.

Der zur Implantation mit Zement vorgesehene Schaft 5* unterscheidet sich in der Gestaltung der Mantelfläche sowie in seiner Weite. Die Mantelfläche ist nicht mit einer Riffelung versehen, sondern mit einigen wenigen Furchen 59*. Wie in Figur8b dargestellt ist, sind vorzugsweise drei Furchen vorgesehen, und zwar äquidistant am Umfang der Mantelfläche 53 verteilt mit einem Winkelabstand von 120°. In Bezug auf die Weite ist der zur Implantation mit Zement vorgesehene Schaft 5* zumindest im Bereich der Mantelfläche 53 um ein Untermaß 58 reduziert. Das Untermaß 58 steht hierbei für die Stärke eines Zementmantels, mit dem der Schaft 5* in den medullären Kanal des Femurs 93 zu verankern ist. Als Beispiel ist in Figur 6b eine Stärke des Zementmantels von 1 mm dargestellt, entsprechend einem Abstand zwischen der gestrichelten und der strichpunktierten Linie. Ferner ist für den Schaft 5* gerade nicht eine Befestigung mittels Presssitz vorgesehen, sodass er ferner bezüglich seiner Weite um das Presssitzmaß 57 reduziert ist. Diese Reduzierung bietet den Vorteil, dass ein und dieselbe Raspel 13 zur Schaffung der benötigten Kavität verwendet werden kann, und zwar unabhängig davon, ob ein Schaft zur zementierten Implantation 5* oder ein Schaft zur zementfreien Implantation 5 schließlich verwendet wird. Somit kann für jede Schaftgröße des Satzes eine einheitliche Raspel verwendet werden, unabhängig von der Befestigungsart.

Der Schaft 5 ist an dem Gelenkelement 4 angeordnet mittels des Adapters 6. Der Adapter 6 ist als Doppelkonus ausgeführt mit einem proximalen Konus 61 und einem distalen Konus 62, die über einen eingeschnittenen Bereich 60 einstückig verbunden sind. Der Konus 61 ist für eine Konusverbindung in einer entsprechenden Aufnahme 56 am distalen Ende des Schafts einzustecken, und entsprechend ist der distale Konus 62 in eine entsprechende Aufnahme einer Konusverbindung am Koppelstück 43 des Gelenkelements einzustecken. Der Adapter ermöglicht eine weitgehend freie Winkelverstellbarkeit zwischen Schaft 5 und Gelenkelement 4, und durch Zusammenstecken der Konusverbindungen mittels Adapter 6 wird diese Winkelposition arretiert. Ferner ist eine Sicherungsschraube 65 optional vorgesehen, welche den Adapter 6 schaftseitig sichert. Entsprechend kann eine gelenkseitige Sicherung vorgesehen sein (nicht dargestellt). Eine Darstellung der Winkelvariabilität zwischen Schaft 5 und Gelenkelement 4 mittels Adapter 6 ist in Figur 11a dargestellt. Wie durch den Doppelpfeil symbolisiert ist, kann die Winkelposition des Schafts 5 frei verändert werden. In einer Detaildarstellung in Figur 11b ist der Übergang zwischen dem Schaft 5 vor dem Adapter 6 zu erkennen, wobei zur Visualisierung einer Winkelposition eine Winkelmarkierung 85 am Schaft 5 angebracht ist.

Die Schäfte 5 sind in den verschiedenen Größen mit unterschiedlicher Länge verfügbar. So gibt es normal lange Schäfte, wie in Figur 10b dargestellt, kurze Schäfte 5', wie in Figur 10a dargestellt, und lange Schäfte 5", wie in Figur 10c dargestellt. Beispielsweise können die kurzen Schäfte 30 mm kürzer und die lange Schäfte 30 mm länger sein als der normal lange Schaft 5. Zweckmäßigerweise sind auch die Adapter 6 in verschiedenen Längen verfügbar, wobei die Länge der Adapter um ein geringeres Maß variiert als die Länge der Schäfte 5. Beispielsweise kann ein kurzer Adapter 6' um 5 oder 10 mm kürzer sein als ein normal langer Adapter 6 bzw. ein langer Adapter 6" kann um 5 oder 10 mm länger sein als ein normaler Adapter 6. Damit kann durch Verwenden eines geeigneten Adapters eine Feineinstellung der Länge erreicht werden, zusätzlich zu der bereits beschriebenen Funktion der Winkelverstellbarkeit und -sicherung.

In Figur 12 ist eine kombinierte Tiefenmesseinrichtung 7 und Winkelmesseinrichtung 8 dargestellt. Sie umfasst eine etwa trapezförmige Grundplatte 70 mit einer zentralen Öffnung 74. Durch diese Öffnung 74 kann ein Schaft eines Implantationsinstruments, insbesondere ein Schaft 14 der Raspel 13, oder eines Bohrers gesteckt werden. Dieser Schaft ist mit Markierungen 75 an einer definierten Stelle versehen. Die Tiefenmesseinrichtung 7 weist einen halbschalenartigen Aufsatz 72 auf, der die Öffnung 74 halbseitig brandet. An einer Oberseite des Aufsatzes 72 ist eine Tiefenmarkierung 73 angeordnet. An einer Rückseite 77 der Grundplatte 70 ist eine abgewinkelte Anlagefläche ausgebildet. Diese wird bei der Implantation auf den Schaft 14 der in der geschaffenen Kavität im Femur 93 steckenden Raspel 13 aufgesteckt und zur Anlage an eine Endfläche am distalen Ende des Femurs 93 gebracht. Damit nimmt die Grundplatte 70 eine definierte Lage zum Femur 93 ein. Es kann dann die Tiefe der Raspel 13 in der Kavität im Femur 93 abgelesen werden mittels der Markierung 75 am Schaft 14 der Raspel 13 bezogen auf die Tiefenmarkierung 73 an der Tiefenmesseinrichtung 7.

Entsprechend ist die Winkelmesseinrichtung aufgebaut. Sie nutzt dieselbe Grundplatte 70. Dazu ist eine Winkelskala 80 vorgesehen. Sie ist ebenfalls die Öffnung 74 berandend angeordnet, und zwar an deren oberen Ende. Ferner ist ein Indikator 82 vorgesehen, der als Stanzöffnung ausgeführt sein kann. Dieser kennzeichnet die Richtung einer Verdrehung, nämlich entweder hin zu dem Indikator 42 oder weg davon (als Ersatz für perspektivenabhängige und damit verwechslungsträchtige Links- bzw. Rechtsdrehungsangaben). Die Winkelskala 82 wirkt zusammen mit einer Markierungsreferenz 81 am Schaft 14 (siehe Figur 13). Man macht sich hierbei die Tatsache zunutze, dass die Raspel 13 in derselben Weise gekrümmt ist wie der Schaft 5. Damit wird sich der Schaft 5 in der von der Raspel 13 geschaffenen Kavität genauso ausrichten wie die Raspel 13. Damit kann die Raspel 13 als eine Art Probeimplantat verwendet werden. Jedoch kann genauso gut ein eigenständiges Problemimplantat vorgesehen sein. Mittels der Winkelskala 80 kann nun die Winkellage der Raspel 13 in der Kavität im Femur 93 bestimmt werden anhand der Markierung 81 auf dem Schaft 14 und dem Indikator 82. Mit den so gewonnenen Angaben für Tiefe- und Winkelposition kann der Schaft 5 in der korrekten Winkelposition an dem Gelenkelement 4 montiert werden und die Prothese in die mit korrekter Tiefe geschaffene Kavität in den Femur 93 eingesetzt werden.

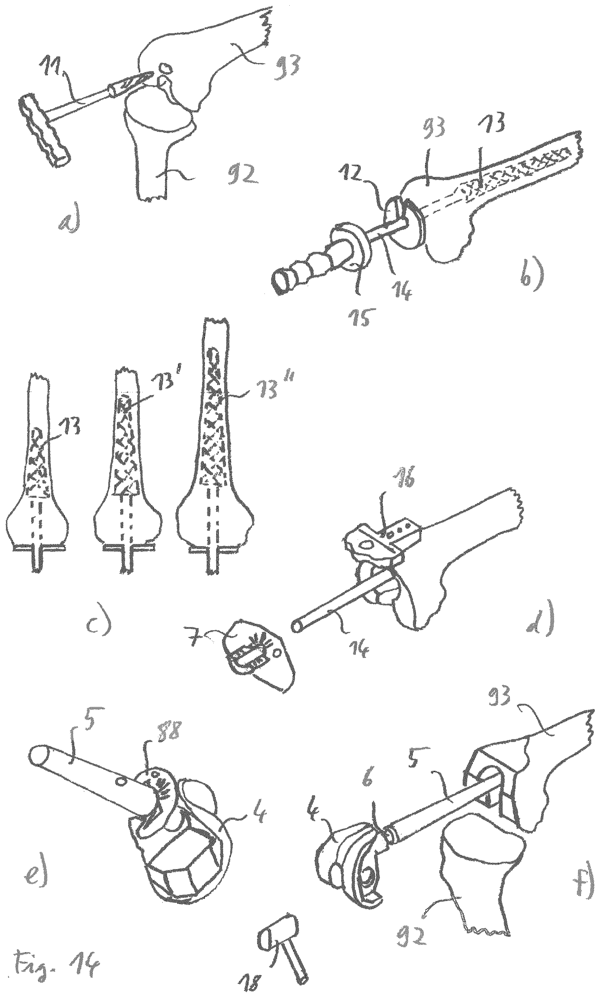

Die einzelnen Schritte bei der Implantation sind in Figur 14 dargestellt. In einem ersten Schritt 14a wird mittels eines Pfriems bzw. eines Bohrers 11 ein Zugang zum medullären Kanal in Femur 93 eröffnet und initial aufgebohrt. Mittels der Raspel 13 wird der medulläre Kanal ausgeweitet und somit die Kavität zur Aufnahme des Schafts 5 geschaffen. Zur Einstellung einer bestimmten Tiefe ist zweckmäßigerweise ein Anschlagteller 12 vorgesehen, der auf den Schaft 14 der Raspel 13 aufgesteckt ist (siehe Figur 14b). Damit kann in Verbindung mit einer entsprechenden Verdickung 15 am Schaft 14 der Raspel 13 sichergestellt werden, dass die Kavität nicht über eine bestimmte Tiefe hinaus aufgeweitet wird. Dann wird in der an sich bekannten Weise der Markraum sukzessive aufgeweitet, bis ein Kortikaliskontakt im medullären Kanal erreicht wird. Zweckmäßigerweise stehen Raspeln 13 mit verschiedenen Längen zur Verfügung; damit kann dann, wenn der Sitz mit der kleinsten Raspel nicht ausreichend fest sein sollte, eine Raspel der gleichen Größe (Weite) aber mit größerer Länge gewählt werden, um so einen sicheren Kortikaliskontakt im medullären Kanal herzustellen. Derartige Raspeln gleicher Weite (Größe) aber unterschiedlicher Länge sind als Raspel 13' und Raspel 13" in Figur 14c dargestellt.

Es können dann in an sich bekannter Weise Lehren angesetzt werden, von denen eine beispielhaft als Lehre 16 dargestellt ist in Figur 14d. Es werden in ebenfalls an sich bekannter Weise dann die erforderlichen Schnitte am distalen Ende des Femurs vorgenommen. Um schließlich die erforderliche Adapterlänge zu bestimmen, wird die Tiefenmesseinrichtung 7 verwendet. Sie wird auf den Schaft 14 aufgesteckt und es wird in der vorstehend beschriebenen Weise die Tiefe gemessen. Je nach Tiefe kann damit ein Adapter 6 passender Länge ausgewählt werden. Damit wird eine Feineinstellung der Tiefe realisiert. Ferner kann in der beschriebenen Weise eine Winkelposition der Kavität und damit des anzusetzenden Schafts 5 im Femur 93 bestimmt werden. Mittels einer Ausrichtlehre 88, die am Übergang zwischen Schaft und Gelenkelement der Femurkomponente 3 temporär angeordnet wird (vorzugsweise wird hierbei eine gesonderte Probeprothese verwendet), wird ein Rotationswinkel zwischen Schaft 5 und Gelenkelement 4 eingestellt (siehe Figur 14e). Dieser Winkel wird schließlich auch verwendet, um so den Schaft 5 passend auf den Adapter 6 aufzustecken und mittels der Konusverbindung zu sichern. Die femorale Komponente 3 mit Gelenkelement und 4 und Schaft 5 ist somit in Länge und (Rotations-)Winkel korrekt eingestellt. Sie kann dann mittels eines (symbolisch dargestellten) Einsatzwerkzeugs 18 an die vorbereitete Stelle am distalen Ende des Femurs 93 implantiert werden (siehe Figur 14f).

## Patentansprüche

1. Kniegelenkendoprothesen-Satz umfassend modulare Kniegelenkendoprothesen in verschiedenen Größen, wobei eine jeweilige Kniegelenkendoprothese umfasst eine tibiale Komponente (2) zur Verankerung an einem proximalen Ende der Tibia, eine femorale Komponente (3) zur Verankerung an einem distalen Ende des Femurs, wobei die femorale Komponente (3) umfasst ein Gelenkelement zum gelenkigen Zusammenwirken mit der tibialen Komponente sowie einen Schaft (5) ausgebildet zur Verankerung in einem Medullärkanal des Femurs, wobei der Satz Schäfte in verschiedenen Größen umfasst, wobei bei der femoralen Komponente (3) eine Form der Schäfte so gewählt ist, dass sie sich von einem distalen, dem Gelenkelement zugewandten Ende (51) zu einem proximalen, freien Schaftende (52) hin verjüngt und an dem distalen Ende (51) mit einem ovalen Querschnitt und an dem proximalen, freien Schaftende (52) mit einem runden Querschnitt ausgeführt ist, wobei eine durch den ovalen Querschnitt bestimmte Ovalität bezüglich ihres Ovalitätsgrads mit zunehmender Größe der Schäfte ansteigt, wobei der Ovalitätsgrad der Ovalität bestimmt ist durch ein Verhältnis von ihrer längeren zu ihrer kürzeren Achse.

2. Kniegelenkendoprothesen-Satz nach Anspruch 1, wobei der ovale Querschnitt (55) eine solche Ovalität aufweist, deren größere Achse (55b) in ML-Richtung und deren kleinere Achse (55a) in AP-Richtung liegt, wobei vorzugsweise ein Verhältnis der langen Achse zur kurzen Achse im Bereich zwischen 1,1 und 1,4 liegt, und weiter vorzugsweise die Ovalität (55) ellipsenartig ausgeführt ist.

3. Kniegelenkendoprothesen-Satz nach einem der vorangehenden Ansprüche, wobei die Schäfte jeweils eine Mantelfläche (53) aufweisen, die zur Anlage an eine Innenwandung des medullären Kanals ausgebildet ist.

4. Kniegelenkendoprothesen-Satz nach einem der vorangehenden Ansprüche, wobei die Schäfte jeweils eine Mantelfläche (53) aufweisen, die einem konischen Übergangskörper zwischen einem ovalen, insbesondere ellipsenförmigen, Querschnitt an einem und einem kreisförmigen Querschnitt am anderen Ende entspricht.

5. Kniegelenkendoprothesen-Satz nach einem der vorangehenden Ansprüche, wobei die Schäfte (5) gekrümmt sind, vorzugsweise schwach gekrümmt sind mit einer Krümmung, die einen Krümmungsradius (R) von mindestens 1000 mm aufweist, weiter vorzugsweise im Bereich zwischen 1200 mm und 1800 mm, weiter vorzugsweise zwischen 1400 mm und 1600 mm.

6. Kniegelenkendoprothesen-Satz nach einem der vorangehenden Ansprüche, wobei die Krümmung der Schäfte (5) in AP-Richtung und ML-Richtung verschieden groß ist, wobei vorzugsweise die Schäfte in AP-Richtung stärker gekrümmt sind, weiter vorzugsweise in ML-Richtung ungekrümmt sind.

7. Kniegelenkendoprothesen-Satz nach einem der vorangehenden Ansprüche, wobei die Schäfte (5) als Kurzschäfte ausgeführt sind mit einer Länge von weniger als dem 7-fachen eines äquivalenten Durchmessers am distalen Schaftende, vorzugsweise weniger als das 5-fache, weiter vorzugsweise mehr als das 2-fache.

8. Kniegelenkendoprothesen-Satz nach einem der vorangehenden Ansprüche, wobei das freie Ende (52) der Schäfte domförmig verrundet ausgeführt ist, wobei vorzugsweise das freie Ende (52) rundum verrundet gestaltet ist.

9. Kniegelenkendoprothesen-Satz nach einem der vorangehenden Ansprüche, wobei Adapter (6) vorgesehen sind, die das Gelenkelement (4) mit einem der Schäfte (5) verbinden, wobei vorzugsweise Adapter (6) in verschiedenen Längen vorgesehen sind.

10. Kniegelenkendoprothesen-Satz nach Anspruch 9, wobei die Adapter (6) bezüglich einer relativen Verschiebung zwischen dem Schaft (5) und dem Gelenkelement arretierbar winkelverstellbar sind, vorzugsweise mit einem Doppelkonus und/oder einer Vielfachverzahnung versehen sind.

11. Kniegelenkendoprothesen-Satz nach einem der vorangehenden Ansprüche, wobei sowohl Schäfte (5*) zur Befestigung mittels Zement wie auch Schäfte (5) zur zementfreien Befestigung vorgesehen sind, wobei die Schäfte zur Befestigung mittels Zement jeweils ein vorbestimmtes Untermaß (58) bezogen auf die korrespondierenden Schäfte zur zementfreien Befestigung aufweisen.

12. Kniegelenkendoprothesen-Satz nach Anspruch 11, wobei die Schäfte (5*) zur Befestigung mittels Zement eine glatte Mantelfläche aufweisen oder mit vorzugsweise maximal fünf Furchen (59*) versehen sind, und/oder die Schäfte zur zementfreien Befestigung eine geriffelte Mantelfläche aufweisen.

13. Kniegelenkendoprothesen-Satz nach einem der vorangehenden Ansprüche, wobei die Größen der Schäfte (5) vorzugsweise regelmäßig gestaffelt sind nach dem ML-Maß, und zwar vorzugsweise auf Basis eines Modulmaßes entsprechend einem Größenschritt zwischen unmittelbar aufeinanderfolgenden Größen des Kniegelenkendoprothesen-Satzes.

14. Kniegelenkendoprothesen-Satz nach Anspruch 13, wobei die Größe der Schäfte sich etwa im Bereich 1 zu 2 erstrecken, und wobei das Modulmaß vorzugsweise so gewählt ist, dass sich zwischen 8 und 14, weiter vorzugsweise zwischen 10 und 12, verschiedene Größen ergeben.

## Claims

1. Knee joint endoprosthesis set comprising modular knee joint endoprostheses in different sizes, each knee joint endoprosthesis comprising a tibial component (2) for anchoring at a proximal end of the tibia, a femoral component (3) for anchoring at a distal end of the femur, the femoral component (3) comprising a joint element for articulated cooperation with the tibial component and comprising a shaft (5) for anchoring in a medullary canal of the femur, the set comprising shafts in different sizes,
wherein,
in the case of the femoral component (3), a shape of the shafts is chosen such that it tapers from a distal end (51), facing toward the joint element, to a proximal free end (52) of the shaft, and is designed with an oval cross section at the distal end (51) and with a round cross section at the proximal free end (52) of the shaft, wherein an ovality defined by the oval cross section increases, with respect to its degree of ovality, as the size of the shafts increases, wherein the degree of ovality of the ovality is defined by a ratio of its longer axis to its shorter axis.

2. Knee joint endoprosthesis set according to Claim 1, wherein the oval cross section (55) has an ovality of the kind whose major axis (55b) lies in the ML direction and whose minor axis (55a) lies in the AP direction, wherein preferably a ratio of the long axis to the short axis lies in the range of between 1.1 and 1.4, and further preferably the ovality (55) is elliptical.

3. Knee joint endoprosthesis set according to either of the preceding claims, wherein the shafts each have a lateral surface (53) which is designed to bear on an inner wall of the medullary canal.

4. Knee joint endoprosthesis set according to any one of the preceding claims, wherein the shafts each have a lateral surface (53) which corresponds to a conical transition body between an oval, in particular an elliptical, cross section at one end and a circular cross section at the other end.

5. Knee joint endoprosthesis set according to any one of the preceding claims, wherein the shafts (5) are curved, preferably weakly curved with a curvature that has a radius of curvature (R) of at least 1000 mm, further preferably in the range of between 1200 mm and 1800 mm, further preferably of between 1400 mm and 1600 mm.

6. Knee joint endoprosthesis set according to any one of the preceding claims, wherein the curvature of the shafts (5) in the AP direction and ML direction is different, wherein preferably the shafts are more strongly curved in the AP direction, further preferably not curved in the ML direction.

7. Knee joint endoprosthesis set according to any one of the preceding claims, wherein the shafts (5) are designed as short shafts with a length of less than 7 times an equivalent diameter at the distal shaft end, preferably less than 5 times, further preferably more than 2 times.

8. Knee joint endoprosthesis set according to any one of the preceding claims, wherein the free end (52) of the shafts has a rounded dome shape, wherein preferably the free end (52) is rounded all the way around.

9. Knee joint endoprosthesis set according to any one of the preceding claims, wherein adapters (6) are provided which connect the joint element (4) to one of the shafts (5), wherein adapters (6) are preferably provided in different lengths.

10. Knee joint endoprosthesis set according to Claim 9, wherein the adapters (6) can be locked at an adjustable angle with respect to a relative displacement between the shaft (5) and the joint element, preferably being provided with a double cone and/or multiple teeth.

11. Knee joint endoprosthesis set according to any one of the preceding claims, wherein shafts (5*) for fastening by means of cement and shafts (5) for cementless fastening are provided, wherein the shafts for fastening by means of cement each have a predetermined undersize (58) relative to the corresponding shafts for cementless fastening.

12. Knee joint endoprosthesis set according to Claim 11, wherein the shafts (5*) for fastening by means of cement have a smooth lateral surface or are provided with preferably a maximum of five furrows (59*), and/or the shafts for cementless fastening have a corrugated lateral surface.

13. Knee joint endoprosthesis set according to any one of the preceding claims, wherein the sizes of the shafts (5) are preferably graded regularly according to the ML dimension, specifically preferably on the basis of a module dimension corresponding to a size step between directly successive sizes of the knee joint endoprosthesis set.

14. Knee joint endoprosthesis set according to Claim 13, wherein the size of the shafts extends approximately in the range of 1 to 2, and the module dimension is preferably chosen such that there are between 8 and 14, further preferably between 10 and 12, different sizes.

## Revendications

1. Jeux d'endoprothèses pour l'articulation du genou comprenant des endoprothèses modulaires pour l'articulation du genou en différentes tailles, dans lequel une endoprothèse respective pour l'articulation du genou comprend un composant tibial (2) pour l'ancrage à une extrémité proximale du tibia, un composant fémoral (3) pour l'ancrage à une extrémité distale du fémur, le composant fémoral (3) comprenant un élément articulé pour l'interaction articulée avec le composant tibial ainsi qu'une tige (5) réalisée pour l'ancrage dans un canal médullaire du fémur, le jeu comprenant des tiges en différentes tailles,
dans lequel
pour le composant fémoral (3), une forme des tiges est choisie de manière telle qu'elle se rétrécit à partir d'une extrémité (51) distale, orientée vers l'élément d'articulation, vers une extrémité (52) de tige proximale libre et est réalisée en l'extrémité (51) distale avec une section transversale ovale et en l'extrémité (52) de tige proximale libre avec une section transversale ronde, une ovalité déterminée par la section transversale ovale augmentant par rapport à son degré d'ovalité avec une taille croissante des tiges, le degré d'ovalité de l'ovalité étant déterminé par un rapport de son axe le plus long à son axe le plus court.

2. Jeu d'endoprothèses pour l'articulation du genou selon la revendication 1, dans lequel la section transversale ovale (55) présente une ovalité dont le plus grand axe (55b) se situe dans le sens ML et dont le plus petit axe (55a) se situe dans le sens AP, un rapport de l'axe le plus long à l'axe le plus court étant de préférence situé dans la plage entre 1,1 et 1,4 et l'ovalité (55) étant plus préférablement réalisée sous forme d'ellipse.

3. Jeu d'endoprothèses pour l'articulation du genou selon l'une des revendications précédentes, dans lequel les tiges présentent à chaque fois une surface d'enveloppe (53) qui est réalisée pour venir en butée contre une paroi interne du canal médullaire.

4. Jeu d'endoprothèses pour l'articulation du genou selon l'une des revendications précédentes, dans lequel les tiges présentent à chaque fois une surface d'enveloppe (53) qui correspond à un corps de transition conique entre une section transversale ovale, en particulier en forme d'ellipse, en une extrémité et une section transversale circulaire en l'autre extrémité.

5. Jeu d'endoprothèses pour l'articulation du genou selon l'une des revendications précédentes, dans lequel les tiges (5) sont incurvées, de préférence faiblement incurvées avec une courbure qui présente un rayon de courbure (R) d'au moins 1000 mm, plus préférablement dans la plage entre 1200 mm et 1800 mm, plus préférablement entre 1400 mm et 1600 mm.

6. Jeu d'endoprothèses pour l'articulation du genou selon l'une des revendications précédentes, dans lequel la courbure des tiges (5) dans le sens AP et dans le sens ML est différente, les tiges étant de préférence plus fortement incurvées dans le sens AP et étant plus préférablement non incurvées dans le sens ML.

7. Jeu d'endoprothèses de l'articulation du genou selon l'une des revendications précédentes, dans lequel les tiges (5) sont exécutées sous forme de tiges courtes présentant une longueur représentant moins de 7 fois, de préférence moins de 5 fois, plus préférablement plus de 2 fois un diamètre équivalent au niveau de l'extrémité distale de tiges.

8. Jeu d'endoprothèses pour l'articulation du genou selon l'une des revendications précédentes, dans lequel l'extrémité libre (52) des tiges est exécuté de manière arrondie sous forme de dôme, l'extrémité libre (52) étant de préférence configurée de manière entièrement arrondie.

9. Jeu d'endoprothèses pour l'articulation du genou selon l'une des revendications précédentes, dans lequel des adaptateurs (6) sont prévus, qui relient l'élément articulé (4) à l'une des tiges (5), les adaptateurs (6) étant de préférence prévus en différentes longueurs.

10. Jeu d'endoprothèses pour l'articulation du genou selon la revendication 9, dans lequel les adaptateurs (6) peuvent être réglés de manière angulaire et bloquée par rapport un déplacement relatif entre la tige (5) et l'élément articulé, de préférence à l'aide d'un double cône et/ou d'une denture multiple.

11. Jeu d'endoprothèses pour l'articulation du genou selon l'une des revendications précédentes, dans lequel des tiges (5*) pour la fixation au moyen d'un ciment ainsi que des tiges (5) pour la fixation sans ciment sont prévues, les tiges pour la fixation au moyen d'un ciment présentant à chaque fois une sous-dimension (58) prédéfinie par rapport aux tiges correspondantes pour la fixation sans ciment.

12. Jeu d'endoprothèses pour l'articulation du genou selon la revendication 11, dans lequel les tiges (5*) pour la fixation au moyen d'un ciment présentent une surface d'enveloppe lisse ou sont pourvues de préférence d'au maximum cinq rainures (59*) et/ou les tiges pour la fixation sans ciment présentent une surface d'enveloppe striée.

13. Jeu d'endoprothèses pour l'articulation du genou selon l'une des revendications précédentes, dans lequel les tailles des tiges (5) sont de préférence échelonnées de manière régulière selon la dimension ML et de préférence sur la base d'une dimension de module correspondant à un pas de taille entre des tailles directement consécutives du jeu d'endoprothèses pour l'articulation du genou.

14. Jeu d'endoprothèses pour l'articulation du genou selon la revendication 13, dans lequel les tailles des tiges s'étendent environ dans la plage de 1 à 2 et dans lequel la dimension modulaire est de préférence choisie de telle sorte qu'on obtient entre 8 et 14, plus préférablement entre 10 et 12 tailles différentes.
